# EUROPÄISCHE PATENTSCHRIFT

(11) **EP 0 602 376 B1**
(45) Veröffentlichungstag und Bekanntmachung des Hinweises auf die Patenterteilung: **23.04.1997**
(21) Anmeldenummer: 93117979.0
(22) Anmeldetag: 05.11.1993
(51) Int. Cl.: C07C 311/53, G03F 7/004

(54) **N,N-Disubstituierte Sulfonamide und damit hergestelltes strahlungsempfindliches Gemisch**
N,N-Disubstituted sulfonamides and radiation-sensitive composition produced therewith
Sulfonamides disubstitués et composition sensible aux radiations obtenue par ces derniers

(30) Priorität: 14.12.1992 DE 4242051
(43) Veröffentlichungstag der Anmeldung: 22.06.1994
(73) Patentinhaber: HOECHST AKTIENGESELLSCHAFT, 65926 Frankfurt am Main (DE)
(72) Erfinder: Eichhorn, Mathias, Dr., D-65527 Niedernhausen (DE); Buhr, Gerhard, Dr., D61462 Königstein (DE)

(56) Entgegenhaltungen:
- FR-A- 2 407 668
- US-A- 3 779 778
- US-A- 3 933 894
- US-A- 3 971 650
- ACTA CHEMICA SCANDINAVICA, SERIES B - ORGANIC CHEMISTRY AND BIOCHEMISTRY, Bd. 40, Nr. 9, 1986, Copenhagen, DK, Seite 745-750, L. GREHN et al: "A simple method for tert-butoxycarbonylation of amides"
- TETRAHEDRON LETTERS, Bd. 30, Nr. 42, 1989, Oxford, GB, Seite 5709-5712 J.R. HENRY et al: "Mitsunobu reactions of N-alkyl and N-acyl sulphonamides - an efficient route to protected amines"
- JOURNAL OF MEDICINAL CHEMISTRY, Bd. 35, Nr. 20, 2. Oktober 1992, Washington, DC, US, Seite 3641-3647, M.J.C. LEE et al: "N-Hydroxylated derivatives of chlorpropamide and its analogues as inhibitors of aldehyde dehydrogenase in vivo"
- JOURNAL OF MEDICINAL CHEMISTRY, Bd. 32, Nr. 6, Juni 1989, Waschington, DC, US, Seite 1335-1340, H.T. NAGASAWA et al: "N1-Alkyl-substituted derivatives of chlorpropamide as inhibitors of aldehyde dehydrogenase"
- SYNTHETIC COMMUNICATIONS, Bd. 20, Nr. 14, 1990, New York, US, Seite 2083-2090, I. ATANASOVA et al: "alpha,alpha,alpha-Trichlormethylcarbonyl compounds as acylating reagents of amides"
- CHEMICAL ABSTRACTS, Bd. 102, Nr. 13, 1. April 1985, Columbus, Ohio, US; Zusammenfassung Nr. 113079a, Seite 672; & JP-A-59 204 165
- CHEMICAL ABSTRACTS, Bd. 93, Nr. 19, 10. November 1980, Columbus, Ohio, US; Zusammenfassung Nr. 181032a, Seite 191; & JP-A-50 089 208
- CHEMICAL ABSTRACTS, Bd. 91, Nr. 25, 17. Dezember 1979, Columbus, Ohio, US; E. TAKEUCHI et al: "Sulfamoylphenylcarbonates", Zusammenfassung Nr. 211082v, Seite 651; & JP-A-54 061 148
- CHEMICAL ABSTRACTS, Bd. 84, Nr. 25, 21. Juni 1976, Columbus, Ohio, US; H. YOSHITAKE et al: "2-Nitrobenzenesulfonamides as herbicides", 175167w, Seite 176; & JP-A-51 015 321
- CHEMICAL ABSTRACTS, Bd. 88, Nr. 7, 13. Februar 1978, Columbus, Ohio, US; K. SATO et al: "Fungicides", Zusammenfassung Nr. 46376x, Seite 179; & JP-A-52 070 020
- CHEMICAL ABSTRACTS, Bd. 72, Nr. 15, 13. April 1970, Columbus, Ohio, US; Zusammenfassung Nr. 78623m, L.P. GLUSHKO et al: "Sulphanilides. XXIII. Isopropyl esters of N-arylsulphonyl-N-phenylcarbamic acids", Seite 356

## Beschreibung

Die Erfindung betrifft N,N-disubstituierte Sulfonamide und ein positiv-arbeitendes strahlungsempfindliches Gemisch, das
a) eine unter der Einwirkung aktinischer Strahlung Säure bildende Verbindung,
b) eine säurespaltbare Verbindung, deren Spaltprodukte in einem wäßrig-alkalischen Entwickler eine höhere Löslichkeit Zeigen als die Ausgangsverbindung, und
c) ein in Wasser unlösliches, in wäßrig-alkalischen Lösungen dagegen lösliches, zumindest quellbares polymeres Bindemittel
enthält. Es eignet sich besonders für ein Aufzeichnungsmaterial aus einem Träger und einer strahlungsempfindlichen Schicht zur Herstellung von Offsetdruckplatten und Photoresists.

Positiv arbeitende strahlungsempfindliche Aufzeichnungsschichten, d. h. Schichten, deren Löslichkeit in den bestrahlten Bereichen größer ist als in den unbestrahlten, sind bekannt. Als lichtempfindliche Komponente in solchen Schichten haben sich insbesondere ortho-Naphthochinondiazide durchgesetzt. Die Lichtempfindlichkeit dieser Schichten ist jedoch meist unbefriedigend.

Die sogenannten "chemisch verstärkten" Gemische zeigen demgegenüber eine höhere Lichtempfindlichkeit, da die Quantenausbeute größer als 1 ist. Positiv arbeitende, "chemisch verstärkte" Gemische enthalten in der Regel eine säurebildende und eine säurespaltbare Komponente, deren Spaltprodukte in wäßrig-alkalischen Entwicklern eine höhere Löslichkeit als die Ursprungsverbindung besitzen.

Als säurespaltbare Verbindungen wurden bisher monomere und polymere Acetale und O,N-Acetale, die als Hydroxyl- oder Aminkomponente aromatische Verbindungen enthalten (US-A 3 779 778), sowie Orthoester und Amidacetale (DE-B 26 10 842 = US-A 4 101 323) verwendet. Strahlungsempfindliche positiv arbeitende Gemische erhält man auch unter Verwendung von polymeren Orthoestern (EP-B 0 022 571 = US-A 4 311 782), polymeren aliphatischen Acetalen (DE-A 27 18 254 = US-A 4 247 611), Enolethern (EP-B 0 006 627 = US-A 4 248 957) sowie N-Acyliminocarbonaten (EP-B 0 006 626 = US-A 4 250 247). Derartige Gemische benötigen zur Auslösung der Spaltreaktion neben der photochemisch erzeugten Säure auch Wasser, was zu Problemen in der praktischen Verwendbarkeit führt. Außerdem sind viele der genannten Verbindungen nur schwer zugänglich.

Ein positiv arbeitendes strahlungsempfindliches Gemisch, das eine Verbindung, die bei Bestrahlung Säure hervorbringt, und ein Polymer mit seitenständigen, säurelabilen tert.-Butoxycarbonyl- oder tert.-Butoxycarbonyloxy-Gruppen enthält, ist in der EP-A 0 102 450 und in der EP-A 0 366 590 beschrieben. Ein ähnliches Gemisch, das jedoch anstelle der genannten Polymere niedermolekulare Verbindungen mit säurelabilen Gruppen enthält, ist in der EP-A 0 249 139 offenbart. Als säurelabile Gruppen in den niedermolekularen Verbindungen, die allgemein ein Molekulargewicht von weniger als 1.000 haben, sind insbesondere tert.-Butoxy-, tert.-Butoxycarbonyl-, tert.-Butoxycarbonyloxy-, 1-Methyl-1-phenyl-ethoxycarbonyl- und Trimethylsilanyloxy-Gruppen offenbart. Solche Systeme benötigen zwar kein Wasser für die Auslösung der Spaltreaktion, sind aber auch nicht frei von Nachteilen: Sie zeigen z. B. einen relativ hohen "Dunkelabtrag", d. h. die Löslichkeit der strahlungsempfindlichen Schicht in einem Entwickler ist auch in den unbelichteten Bereichen relativ hoch, was zu einer schlechten Differenzierung zwischen belichteten und unbelichteten Bereichen führt.

Aufgabe der Erfindung war es, säurespaltbare Verbindungen bereitzustellen, die sich besonders gut für ein positiv-arbeitendes Gemisch mit einer hohen Empfindlichkeit für aktinische Strahlung, insbesondere kurzwellige aktinische Strahlung, eignen und dabei einfach und kostengünstig herstellbar sind.

Erfindungsgemäß wird diese Aufgabe gelöst durch die Bereitstellung von N,N-disubstituierten Sulfonamiden der allgemeinen Formeln R¹[-N(CO-OR²)-SO₂-R³]ₙ (I) oder R¹[-SO₂-N(CO-OR²)-R³]ₙ (II) worin
- R¹: im Fall n=1, ein (C₁-C₂₀)Alkyl-, (C₃-C₁₀)Cycloalkyl-, (C₆-C₁₄)Aryl- oder (C₇-C₂₀)Aralkylrest, wobei in den Alkyl enthaltenden Resten einzelne Methylengruppen durch Heteroatome ersetzt sein können, und
im Fall n = 2, 3 oder 4, der n-wertige Rest eines (C₁-C₂₀)Alkans oder (C₅-C₇)Cycloalkans, wobei in den n-wertigen (C₃-C₂₀)Alkanresten einzelne Methylengruppen durch Heteroatome, durch (C₅-C₇)-Cycloalkylenreste oder durch Phenylen ersetzt sein können, oder der n-wertige Rest eines nicht-kondensierten oder kondensierten ein- oder mehrkerniger (C₆-C₁₈)Aromaten,
- R²: ein (C₃-C₁₁)Alkyl-, (C₃-C₁₁)Alkenyl- oder (C₇-C₁₁)-Aralkylrest,
- R³: ein unsubstituierter oder substituierter (C₁-C₆)Alkyl-, (C₃-C₆)Cycloalkyl-, (C₆-C₁₄)Aryl- oder (C₇-C₂₀)Aralkylrest
ist, und
- n: eine ganze Zahl von 1 bis 4 bedeutet.

Die Verbindungen der Formeln I und II mit n = 2, 3 oder 4 sind neu und Teil der vorliegenden Erfindung.

Im Rest R¹ sind vorzugsweise nicht mehr als drei Methylengruppen durch Heteroatome, Cycloalkylen und/oder Phenylen ersetzt. Als Heteroatom kommt insbesondere Sauerstoff in Betracht. Verbindungen mit n = 1 oder 2 sind allgemein bevorzugt. Im Fall n = 2 hat ein bevorzugter Rest R¹ die Formel -A-X-B-, worin X eine C-C-Einfachbindung, eine Methylengruppe oder ein Sauerstoffatom darstellt und A und B, unabhängig voneinander, für Arylen, insbesondere ortho-, meta- oder para-Phenylen, oder für Cycloalkandiyl, insbesondere Cyclohexandiyl, stehen.

Die Verbindungen der Formeln I oder II lassen sich aus N-monosubstituierten Sulfonamiden herstellen, indem man diese in Gegenwart einer katalytischen Menge einer organischen Base, wie 4-Dimethylaminopyridin, mit aktivierten Kohlensäureestern, die die Gruppierung -OR² als Alkoholkomponente enthalten, umsetzt.

Die N-monosubstituierten Sulfonamide sind nach den bekannten Verfahren aus Sulfonsäuren und primären Aminen herstellbar. Für die Herstellung der Verbindungen der Formeln I und II mit n = 1 werden Monoamine verwendet, für die der Verbindungen der Formel I mit n = 2, 3 oder 4 Diamine, Triamine oder Tetraamine. Die Sulfonsäuren werden nicht als solche in die Reaktion eingesetzt, sondern in einer reaktionsfähigeren, sogenannten "aktivierten" Form. Hierzu zählen besonders die Sulfonylhalogenide, speziell die Sulfonylchloride.

Bevorzugte Monoamine sind geradkettige oder verzweigte Alkylamine mit 1 bis 12 Kohlenstoffatomen, besonders bevorzugt 1 bis 6 Kohlenstoffatomen, wie Methylamin, Ethylamin, Propylamin, Isopropylamin, Butylamin, sec.-Butylamin, Isobutylamin, tert.-Butylamin, Pentylamin, 1-Methyl-butylamin, 2-Methyl-butylamin und Hexylamin. Bevorzugt sind daneben auch Cycloalkylamine mit 3 bis 12 Kohlenstoffatomen, besonders bevorzugt 5 bis 8 Kohlenstoffatomen, wie Cyclopentylamin, Cyclohexylamin, Cycloheptylamin und Cyclooctylamin. Von den aromatischen Monoaminen sind solche mit 6 bis 15 Kohlenstoffatomen bevorzugt, deren aromatischer Teil substituiert sein kann, besonders mit Halogenatomen, Alkyl- oder Alkoxygruppen. Beispiele für bevorzugte aromatische Amine sind Anilin, 4-Methyl-anilin, 4-Ethyl-anilin, 4-Methoxy-anilin, 3-Methoxy-anilin, 4-Ethoxy-anilin, 4-Phenoxy-anilin, Naphthylamin, Biphenylamin, 1- und 2-Amino-anthracen und 9-Amino-phenanthren. Von den Aralkylaminen sind solche mit 7 bis 20 Kohlenstoffatomen bevorzugt. Diese können in der gleichen Weise wie die aromatischen Amine substituiert sein. Beispiele hierfür sind Benzylamin, 4-Methoxy-benzylamin, 2,2- und 3,3-Diphenyl-propylamin.

Bevorzugte Diamine sind geradkettige oder verzweigte Alkylendiamine mit 2 bis 20 Kohlenstoffatomen, die auch substituiert sein können. Daneben können auch einzelne Methylengruppen durch Heteroatome, insbesondere Sauerstoff, ersetzt sein. Beispiele hierfür sind Ethylendiamin, Propan-1,3-diamin, Butan-1,3- und -1,4-diamin, Pentan-1,5-diamin, Hexan-1,6-diamin, 4,9-Dioxa-dodecan-1,12-diamin und 4,7,10-Trioxa-tridecan-1,13-diamin. Bevorzugt sind auch Cycloalkylendiamine mit 3 bis 13 Kohlenstoffatomen, wie Cyclopentan-1,2- und -1,3-diamin, Cyclohexan-1,2-, -1,3- und -1,4-diamin und Methylen-bis-cyclohexan-4,4'-diyldiamin. Bevorzugte aromatische Diamine sind solche mit 6 bis 15 Kohlenstoffatomen, wie ortho-, meta- und para-Phenylendiamin, 2-Methyl-paraphenylendiamin, Benzidin, Naphthalin-1,2-, -1,3-, -1,4-, -1,5-, -2,3-, -2,4- und -2,5-diamin, Anthracendiamin, Acridin-3,6-diamin und Phenanthren-9,10-diamin.

Ein Beispiel für ein Tetraamin ist Biphenyltetraamin.

Zur Herstellung der erfindungsgemäßen Verbindungen der Formeln I und II mit n = 1 bevorzugte Sulfonsäuren sind Methansulfonsäure, Trifluormethansulfonsäure, Ethansulfonsäure, Propansulfonsäure, Perfluorpropansulfonsäure, Perfluorbutansulfonsäure, Hexansulfonsäure, Perfluoroctansulfonsäure, Benzolsulfonsäure, Pentafluorbenzolsulfonsäure, para-Toluolsulfonsäure, Naphthalinsulfonsäure, zur Herstellung der Verbindungen der Formel II, n = 2, 3 oder 4, insbesondere Hexandisulfonsäure, Benzoldisulfonsäure, Naphthalindi-, -tri- und -tetrasulfonsäure, Biphenyldisulfonsäure und 4,4'-Oxy-bis-benzolsulfonsäure.

Als "aktivierte" Kohlensäureester werden solche bezeichnet, die eine Acylierung der N-monosubstituierten Sulfonamide mit -CO-OR² bewirken können. Dies sind insbesondere Dialkyldicarbonate (= Pyrokohlensäure-dialkylester). Besonders bevorzugt ist Di-tert.-butyldicarbonat (= O[CO₂-C(CH₃)₃]₂).

Die Umsetzung der N-monosubstituierten Sulfonamide mit den aktivierten Kohlensäureestern erfolgt vorzugsweise in einem Lösemittel, das mit den übrigen Bestandteilen des Gemisches nicht irreversibel reagiert, in Gegenwart von 0,01 bis 10 mol-%, bevorzugt 0,05 bis 2 mol-%, jeweils bezogen auf die molare Menge an N-monosubstituiertem Sulfonamid, einer organischen Base. Diese Base ist bevorzugt ein tertiäres Amin, z. B. ein Dialkylaminopyridin. Geeignete Lösemittel sind insbesondere Tetrahydrofuran, Ethylacetat, Diethylether, Butanon (= Methylethylketon). Es hat sich als zweckmäßig erwiesen, das N-monosubstituierte Sulfonamid und die organische Base in gelöster Form vorzulegen und zu diesem Gemisch den aktivierten Kohlensäureester langsam zuzugeben. Die Reaktion wird im allgemeinen bei einer Temperatur von 0 bis 80 °C, bevorzugt 10 bis 50 °C, durchgeführt. Das Reaktionsprodukt kann dann durch Eingießen des Reaktionsgemisches in Wasser, Abfiltrieren des Niederschlags und Trocknen, oder einfach durch Abziehen der flüchtigen Bestandteile unter vermindertem Druck in ausreichender Reinheit isoliert werden. Falls erforderlich, kann durch Umkristallisieren, Umfällen, Destillieren oder durch ein präparatives chromatographisches Verfahren eine weitere Reinigung erfolgen.

Erfindungsgemäß wird weiterhin ein strahlungsempfindliches Gemisch vorgeschlagen, das
a) eine Verbindung, die unter der Einwirkung aktinischer Strahlung Säure bildet,
b) eine säurespaltbare Verbindung, deren Spaltprodukte in einem wäßrig-alkalischen Entwickler eine höhere Löslichkeit zeigen als die Ausgangsverbindung, und
c) ein in Wasser unlösliches, in einem wäßrig-alkalischen Entwickler dagegen lösliches, zumindest quellbares, polymeres, organisches Bindemittel,
enthält und dadurch gekennzeichnet ist, daß die säurespaltbare Verbindung b) ein N,N-disubstituiertes Sulfonamid der allgemeinen Formel I oder II ist.

Als Verbindungen a), die unter der Einwirkung aktinischer Strahlungs vorzugsweise starke Säuren bilden, sind insbesondere Diazonium-, Phosphonium-, Sulfonium- und Iodoniumsalze, Halogenverbindungen, o-Chinondiazidsulfochloride, -ester und -amide sowie Organometall/Organohalogen-Kombinationen geeignet. Die genannten Diazonium-, Phosphonium-, Sulfonium- und Iodoniumverbindungen werden in der Regel eingesetzt in Form ihrer in organischen Lösemitteln löslichen Salze, insbesondere in Form der Sulfonate, speziell Trifluormethansulfonate, der Tetrafluorborate, Hexafluorphosphate, Hexafluorantimonate oder Hexafluorarsenate. Es können aber auch Halogenide, Ester und Amide von 1,2-Naphthochinon-2-diazid-sulfonsäuren verwendet werden. Die Azidität der beim Bestrahlen von o-Naphthochinondiaziden entstehenden Indencarbonsäuren reicht jedoch meist nur knapp für eine ausreichende bildmäßige Differenzierung aus. Bevorzugt wird daher aus dieser Gruppe das 1,2-Naphthochinon-2-diazid-4-sulfonylchlorid, bei dessen Bestrahlung drei Säurefunktionen gebildet werden, so daß ein relativ großer Verstärkungsfaktor besteht. Schließlich sind als Säurebildner auch organische Halogenverbindungen brauchbar, beispielsweise solche mit mehr als einem Halogenatom an einem Kohlenstoffatom oder an einem aromatischen Ring. Die spektrale Empfindlichkeit dieser halogenhaltigen Verbindungen kann durch an sich bekannte Sensibilisatoren verändert und gesteigert werden. Beispiele für besonders geeignete Säurebildner sind 1,2-Naphthochinon-2-diazid-4-sulfonylchlorid; 4-Dipropylamino-benzoldiazonium-tetrafluorborat, -hexafluorphosphat und -trifluormethansulfonat; 2,5-Diethoxy-4-p-tolylmercapto-benzoldiazonium-tetrafluorborat, -hexafluorphosphat und -trifluormethansulfonat; 4-Anilino-benzoldiazonium-sulfat und 4-Diethylamino-benzoldiazonium-trifluormethansulfonat sowie die in den Beispielen angeführten Verbindungen. Ebenfalls verwendet werden können 4-Methyl-6-trichlormethy1-2-pyron; 4-(3,4,5-Trimethoxystyryl)-6-trichlormethyl-2-pyron; 4-(4-Methoxystyryl)-6-(3,3,3-trichlorpropenyl)-2-pyron; 2-Trichlormethyl-benzimidazol; 2-Tribrommethyl-chinolin-4-on; 2,4-Dimethyl-1-tribromacetyl-benzol; 3-Nitro-1-tribromacetyl-benzol; 4-Dibromacetylbenzoesäure; 1,4-Bis-dibrommethyl-benzol; substituierte 4,6-Bis-trichlormethyl-s-triazine wie 2-(6-Methoxy-naphthalin-2-yl)-, 2-Naphthalin-1-yl-, 2-Naphthalin-2-yl-, 2-[4-(2-Ethoxy-ethyl)-naphthalin-1-yl]-, 2-Benzopyran-3-yl-, 2-Phenanthren-9-yl- und 2-(4-Methoxy-anthracen-1-yl)-4,6-bis-trichlormethyl-s-triazin und Tris-dibrommethyl-s-triazin.

Der Anteil an säurebildender Komponente a) im Gemisch ist je nach Zusammensetzung des Gemisches variierbar. Günstige Ergebnisse werden erhalten mit etwa 0,1 bis 20 Gew.-%, bevorzugt 0,2 bis 10% Gew.-%, jeweils bezogen auf das Gesamtgewicht der Feststoffe im Gemisch. Besonders für Kopierschichten mit einer Dicke von mehr als 10 µm empfiehlt es sich, relativ wenig Säurebildner zu verwenden.

Der Anteil an säurespaltbarer Komponente b) beträgt im allgemeinen 5 bis 50 Gew.-%, bevorzugt 10 bis 30 Gew.-%, jeweils bezogen auf das Gesamtgewicht der Feststoffe im Gemisch.

Neben den erfindungsgemäßen können noch andere säurespaltbare Verbindungen in dem Gemisch vorhanden sein. Dies sind insbesondere Polymere mit tert.-Butoxycarbonyl-Gruppen. Gemische mit solchen weiteren säurespaltbaren Verbindungen sind allgemein jedoch nicht bevorzugt.

Besonders geeignete polymere Bindemittel c) sind Phenolharze, insbesondere Kresol-Formaldehyd-Novolake (Schmelzbereich 105-120°C nach DIN 53181) und Phenol-Formaldehyd-Novolake (Schmelzbereich 110-120°C nach DIN 53181).

Art und Anteil des Bindemittels richten sich nach dem Anwendungszweck. Bevorzugt ist ein Anteil von 30 bis 90 Gew.-%, besonders bevorzugt von 55 bis 85 Gew.-%, jeweils bezogen auf das Gesamtgewicht der Feststoffe im Gemisch.

Bindemittel, deren Alkalilöslichkeit durch Einwirkung von Säure erhöht wird, sind im erfindungsgemäßen Gemisch ebenfalls verwendbar. Solche Bindemittel können z.B. Polyhydroxystyrole sein, deren phenolische OH-Gruppen mit säurelabilen Gruppierungen versehen sind, die die Alkalilöslichkeit herabsetzen. Die erfindungsgemäßen Verbindungen bewirken eine deutliche Verringerung des Dunkelabtrags, ohne die Lichtempfindlichkeit des Gemisches zu beeinträchtigen.

Andere alkalilösliche Harze, wie Copolymere aus Methacrylsäure und Methylmethacrylat, Vinylacetat und Crotonsäure, sowie Maleinsäureanhydrid und Styrol, sind als Bindemittel ebenfalls geeignet.

Zusätzlich können noch zahlreiche andere Harze mitverwendet werden, bevorzugt Vinylpolymere wie Polyvinylacetate, Polyacrylate, Polyvinylether und Polyvinylpyrrolidone, die wiederum durch Comonomere modifiziert sein können. Der günstigste Anteil an diesen Harzen richtet sich nach den anwendungstechnischen Erfordernissen und dem Einfluß auf die Entwicklungsbedingungen. Er beträgt im allgemeinen nicht mehr als 20 Gew.-%, bezogen auf das Gesamtgewicht des Bindemittels.

Um spezielle Erfordernisse, wie Flexibilität, Haftung, oder Glanz zu erfüllen, kann das strahlungsempfindliche Gemisch daneben noch Substanzen wie Polyglykole, Cellulose-Derivate, wie Ethylcellulose, Netzmittel, Farbstoffe und feinteilige Pigmente enthalten. Als Farbstoffe haben sich die Triphenylmethanfarbstoffe, insbesondere in Form ihrer Carbinolbasen, besonders bewährt. Die günstigsten Mengenverhältnisse der Komponenten lassen sich im Einzelfall durch Versuche leicht ermitteln.

Gegenstand der vorliegenden Erfindung ist schließlich auch ein Aufzeichnungsmaterial mit einem Träger und einer strahlungsempfindlichen Schicht aus dem erfindungsgemäßen Gemisch. Das Aufzeichnungsmaterial wird üblicherweise durch Beschichten des Trägers mit einer Lösung des Gemisches hergestellt.

Geeignete Lösemittel für das erfindungsgemäße strahlungsempfindliche Gemisch sind Ketone wie Methylethylketon, chlorierte Kohlenwasserstoffe wie Trichlorethylen und 1,1,1-Trichlorethan, Alkohole wie n-Propanol, Ether wie Tetrahydrofuran, Glykolether wie Ethylenglykolmonoethylether und Ester wie Butylacetat. Es können auch Gemische verwendet werden, die zudem noch für spezielle Zwecke Lösemittel wie Acetonitril, Dioxan oder Dimethylformamid enthalten können. Prinzipiell sind alle Lösemittel verwendbar, die mit den Schichtkomponenten nicht irreversibel reagieren. Sie sind jedoch im Hinblick auf das vorgesehene Beschichtungsverfahren, die Schichtdicke und die Trocknungsvorrichtung auszuwählen. Dünne Schichten bis ca. 5 µm werden für Versuchsmengen vorzugsweise aufgeschleudert. Schichtdicken von mehr als 60 µm sind mit Lösungen bis zu ca. 40 % Feststoffgehalt bei einmaligem Auftrag auf der Schleuder oder mit einer Streichrakel erreichbar. Für beidseitiges Beschichten wird Tauchbeschichtung vorgezogen, wobei schnelles Antrocknen von Vorteil ist, das durch die Verwendung niedrigsiedender Lösemittel erreicht wird. Bandförmige Trägermaterialien können durch Aufsprühen der Beschichtungslösung mit Breitschlitzdüsen oder Antragen mit Walzen, Einzelplatten - wie Zink- und Mehrmetallplatten - durch Gießantrag (curtain coating) beschichtet werden.

Im Vergleich zu anderen Positivschichten, besonders denen auf o-Naphthochinondiazidbasis, ist auch die Herstellung dickerer Schichten möglich, da die Lichtempfindlichkeit der erfindungsgemäßen Gemische verhältnismäßig wenig dickenabhängig ist. Belichtung und Verarbeitung von Schichten einer Dicke von 100 µm und darüber sind möglich.

Bevorzugte Träger für Schichten von mehr als 10 µm Dicke sind Kunststoffolien, die dann als temporäre Träger für Transferschichten dienen. Dafür und für Farbfolien werden Polyesterfolien, z. B. aus Polyethylenterephthalat, bevorzugt. Polyolefinfolien wie Polypropylen sind jedoch ebenfalls geeignet. Als Träger für Schichten von weniger als 10 µm werden meist Metalle verwendet. Zur Herstellung von Offsetdruckplatten kann mechanisch oder elektrochemisch aufgerauhtes und gegebenenfalls anodisiertes Aluminium, das evtl. chemisch (z.B. mit Polyvinylphosphonsäure, Silikaten oder Phosphaten) vorbehandelt ist, verwendet werden. Geeignet sind auch Mehrmetallplatten mit Cu/Cr oder Messing/Cr als oberster Schicht. Für Hochdruckplatten können die erfindungsgemäßen Schichten auf Zink- oder Magnesiumplatten sowie deren handelsübliche mikrokristalline Legierungen für Einstufenätzverfahren aufgetragen werden, außerdem auf ätzbare Kunststoffe wie Polyoxymethylen. Für Tiefdruck- oder Siebdruckformen eignen sich diese Schichten durch ihre gute Haftung und Ätzfestigkeit auf Kupfer- bzw. Nickeloberflächen. Ebenso lassen sie sich als Photoresists und beim Formteilätzen einsetzen.

Schließlich kann die Beschichtung direkt oder durch trockene Schichtübertragung vom temporären Träger erfolgen auf Leiterplattenmaterialien, die aus Isolierplatten mit ein- oder beidseitiger Kupferauflage bestehen, auf Glas- oder Keramikmaterialien, die ggf. haftvermittelnd vorbehandelt sind, und u. a. auf Siliciumscheiben, auf deren Oberfläche sich ggf. eine Nitrid- oder Oxidschicht befindet. Außerdem können Holz, Textilien und Oberflächen vieler Werkstoffe beschichtet werden, die vorzugsweise durch Projektion bebildert werden und resistent gegen die Einwirkung alkalischer Entwickler sind.

Die Beschichtung kann mit den die üblichen Geräten und unter den üblichen Bedingungen getrocknet werden. Sie verträgt Temperaturen um 100 °C, kurzfristig sogar bis zu 120 °C, ohne daß später eine Einbuße an Strahlungsempfindlichkeit beobachtet wird.

Zum Bestrahlen können die üblichen Strahlungsquellen, wie Röhrenlampen, Xenonimpulslampen, metallhalogeniddotierte Quecksilberdampf-Hochdrucklampen und Kohlebogenlampen verwendet werden. Darüber hinaus ist das Bestrahlen in üblichen Projektions- und Vergrößerungsgeräten unter dem Licht der Metallfadenlampen und Kontaktbelichtung mit gewöhnlichen Glühbirnen möglich. Die Bestrahlung kann auch mit dem kohärenten Licht eines Lasers erfolgen. Geeignet sind hier leistungsfähige kurzwellige Laser, beispielsweise Argon-Ionen-Laser, Krypton-Ionen-Laser, Farbstoff-Laser, Helium-Cadmium-Laser sowie Excimer-Laser, die zwischen 193 und 633 nm emittieren. Der Laser wird üblicherweise computergesteuert raster- oder strichartig über die Aufzeichnungsschicht geführt und bestrahlt diese dabei bildmäßig.

Das Bestrahlen mit Elektronenstrahlen ist eine weitere Bebilderungsmöglichkeit. Elektronenstrahlen können das erfindungsgemäße Gemisch wie auch viele andere organische Materialien durchgreifend zersetzen und anschließend vernetzen, so daß ein negatives Bild entsteht, wenn die unbestrahlten Teile durch Lösemittel oder Belichten ohne Vorlage und Entwickeln entfernt werden. Bei geringerer Intensität und/oder höherer Schreibgeschwindigkeit des Elektronenstrahls bewirkt dagegen der Elektronenstrahl eine Differenzierung in Richtung höherer Löslichkeit, d. h. die bestrahlten Schichtpartien können vom Entwickler entfernt werden. Die Wahl der günstigsten Bedingungen kann durch Versuche leicht ermittelt werden.

Die bildmäßig belichtete oder bestrahlte Schicht kann, gegebenenfalls nach einer thermischen Nachbehandlung, mit praktisch den gleichen Entwicklern wie für handelsübliche Naphthochinondiazidschichten und Kopierlacke entfernt werden, bzw. die neuen Schichten können in ihren Kopierbedingungen vorteilhaft auf die bekannten Hilfsmittel, wie Entwickler und programmierte Sprühentwicklungsgeräte, abgestimmt werden. Die wäßrigen Entwicklerlösungen können z. B. Alkaliphosphate, -silikate oder -hydroxide und ferner Netzmittel sowie kleinere Anteile organischer Lösemittel enthalten. In bestimmten Fällen sind anstelle der wäßrig-alkalischen Entwickler auch organische Lösemittel oder Gemische von organischen Lösemitteln mit Wasser als Entwickler brauchbar. Bevorzugt als Entwickler sind jedoch wäßrig-alkalische Lösungen.

Der günstigste Entwickler kann durch Versuche mit der jeweils verwendeten Schicht ermittelt werden. Bei Bedarf kann die Entwicklung mechanisch unterstützt werden. Zur Erhöhung der Widerstandsfähigkeit beim Druck sowie der Resistenz gegen Auswaschmittel, Korrekturmittel und durch UV-Licht härtbare Druckfarben können die entwickelten Platten kurze Zeit auf erhöhte Temperaturen erwärmt werden.

Im folgenden werden Beispiele für die bevorzugten erfindungsgemäßen N,N-disubstituierten Sulfonamide, ihre Synthese und die bevorzugten, unter ihrer Verwendung erhältlichen, lichtempfindlichen Gemische angegeben. Gt steht in den Beispielen für Gewichtsteile.

### Beispiele 1 bis 18

1 mol N-substituiertes Sulfonamid und 0,02 mol 4-Dimethylamino-pyridin werden in 700 ml Ethylacetat oder Tetrahydrofuran gelöst. Bei Raumtemperatur tropft man innerhalb von 30 min eine Lösung von n mol Di-tert-butyldicarbonat in 300 ml Ethylacetat oder Tetrahydrofuran zu der gerührten Lösung. Nach beendeter Reaktion isoliert man das Produkt durch Abziehen des Lösemittels unter vermindertem Druck oder durch Eingießen der Reaktionslösung in Wasser, Absaugen des dabei ausgefallenen Produkts und Trocknen. Man erhält nach diesem Verfahren die in Tabelle 1 aufgeführten N,N-disubstituierten Sulfonamide.

**Tabelle 1**

| Erfindungsgemäße N,N-disubstituierte Sulfonamide | | | | | | |
|---|---|---|---|---|---|---|
| Beispiel | Formel | R¹ | R² | R³ | n | Fp (°C) |
| 1 | I | Methyl | t-Bu | p-Tolyl | 1 | 72- 73 |
| 2 | I | n-Propyl | t-Bu | p-Tolyl | 1 | 43- 44 |
| 3 | I | n-Butyl | t-Bu | p-Tolyl | 1 | 51- 52 |
| 4 | I | Phenyl | t-Bu | p-Tolyl | 1 | 122-123 |
| 5 | I | Phenyl | t-Bu | Methyl | 1 | 109-110 |
| 6 | I | Phenyl | t-Bu | 2-Naphtyl | 1 | 152-153 |
| 7 | I | Phenyl | t-Bu | 1-Naphtyl | 1 | 82- 83 |
| 8 | II | Biphenyl-4,4'-diyl | t-Bu | n-Propyl | 2 | 153-154 |
| 9 | II | Naphthalin-1,5-diyl | t-Bu | Phenyl | 2 | 251-252 |
| 10 | II | Biphenyl-4,4'-diyl | t-Bu | Phenyl | 2 | 223-224 |
| 11 | II | Oxybiphenyl-4,4'-diyl | t-Bu | Benzyl | 2 | 131-132 |
| 12 | II | Biphenyl-4,4'-diyl | t-Bu | Benzyl | 2 | 246-249 |
| 13 | I | Butan-1,4-diyl | t-Bu | p-Tolyl | 2 | 132-133 |
| 14 | I | p-Phenylen | t-Bu | p-Tolyl | 2 | 277-278 |
| 15 | I | Oxybiphenyl-4,4'-diyl | t-Bu | p-Tolyl | 2 | 163 |
| 16 | I | 4,9-Dioxa-dodecan-1,12-diyl | t-Bu | p-Tolyl | 2 | 84- 85 |
| 17 | I | 4,7,10-Trioxa-tridecan-1,13-diyl | t-Bu | p-Tolyl | 2 | Öl |
| 18 | I | Methylen-bis-cyclohexan-4,4'-diyl | t-Bu | p-Tolyl | 2 | 174-175 |

### Beispiel 19

Eine Beschichtungslösung aus
- 6,50 Gt: Novolak,
- 2,50 Gt: jeweils eines der in Tabelle 1 angegebenen N,N-disubstituierten Sulfonamide,
- 0,50 Gt: 4-p-Tolylmercapto-2,5-diethoxy-benzoldiazoniumhexafluorphosphat,
- 0,08 Gt: Kristallviolettbase und
- 175 Gt: Methylethylketon
wird auf Platten aus elektrochemisch aufgerauhtem und anodisiertem Aluminium aufgeschleudert und bei 100 °C im Trockenschrank getrocknet, wobei die Schichtdicke nach dem Trocknen 1,8 bis 2,0 µm beträgt. Die Platten werden mit einer 5-kW-Metallhalogenidlampe, die sich in einem Abstand von 110 cm über der strahlungsempfindlichen Schicht befindet, durch einen Halbtonstufenkeil mit 13 Stufen einer Dichteabstufung von 0,15 bestrahlt, 1 min bei 100 °C im Trockenschrank erwärmt und in einem wäßrig-alkalischen Entwickler folgender Zusammensetzung
- 5,5 Gt: Natriummetasilikat·9 H₂O,
- 3,4 Gt: Trinatriumphosphat·12 H₂O,
- 0,4 Gt: Mononatriumphosphat wasserfrei und
- 90,7 Gt: vollentsalztes Wasser
entwickelt. Es werden einwandfreie, positive Abbilder der Druckvorlage erhalten. Bei Verwendung der Verbindungen 8, 9, 12 oder 16 zeigen die Abbilder eine etwas verminderte Qualität.

### Beispiele 20 bis 27

Eine Platte aus elektrochemisch aufgerauhtem und anodisiertem Aluminium wird mit einer Lösung aus
- 6,50 Gt: Bindemittel (BM),
- 2,50 Gt: N,N-disubstituiertem Sulfonamid,
- 0,50 Gt: 4-p-Tolylmercapto-2,5-diethoxy-benzoldiazoniumhexafluorphosphat,
- 0,08 Gt: Kristallviolettbase und
- 175 Gt: Methylethylketon
schleuderbeschichtet und bei 100 °C im Trockenschrank erhitzt, wobei eine Schichtdicke von 1,9 µm resultiert. Die Platte wird unter einer 5-kW-Metallhalogenidlampe im Abstand von 110 cm durch einen Halbtonstufenkeil mit 13 Stufen einer Dichteabstufung von 0,15 belichtet, 1 min bei 100 °C nacherwärmt und in einem wäßrig-alkalischen Entwickler der im Beispiel 19 angegebenen Zusammensetzung 30 s lang entwickelt. In allen Fällen wird ein positives Abbild der Druckvorlage erhalten. Tabelle 2 gibt ferner an, bei welcher Belichtungszeit die Stufe 4 des Halbtonkeils auf der Platte offen wiedergegeben wird und zeigt damit die deutlich höhere Lichtempfindlichkeit der erfindungsgemäßen lichtempfindlichen Schichten gegenüber bekannten Kopierlacken.

**Tabelle 2**

| Beispiel | Sulfonamid lt. Tab. 1 | Belichtungszeit in s | Bindemittel |
|---|---|---|---|
| 20 | 2 | 40 | Novolak |
| 21 | 4 | 40 | Novolak |
| 22 | 6 | 55 | Novolak |
| 23 | 6 | 40 | Poly-3-methyl-p-hydroxystyrol |
| 24 | 1 | 45 | Copolymer aus Brenzkatchinmonomethacrylat und Styrol |
| 25 | 7 | 65 | Novolak |
| 26 | 17 | 40 | Novolak |
| 27 | 1 | 30 | Novolak |
| Vgl.* | - | 75 | Novolak |

| | | | |
|---|---|---|---|
| * Verwendet wurde eine Offsetdruckplatte ^{(R)}Ozasol P 61 (Hoechst AG) | | | |

### Beispiel 28

Dieses Beispiel zeigt die Eignung der erfindungsgemäßen Gemische für die Herstellung von positiv-arbeitenden Offsetdruckplatten.

Eine Platte aus elektrochemisch aufgerauhtem und anodisiertem Aluminium wird mit einer Lösung aus
- 7,00 Gt: Novolak,
- 2,00 Gt: N-tert.-Butoxycarbonyl-N-phenyl-naphthalin-2-sulfonamid (Beispiel 6 in Tabelle 1),
- 0,50 Gt: 4-p-Tolylmercapto-2,5-diethoxybenzoldiazoniumhexafluorphosphat,
- 0,08 Gt: Kristallviolettbase und
- 175 Gt: Methylethylketon
schleuderbeschichtet und bei 100 °C im Trockenschrank erhitzt, wobei eine Schichtdicke von 1,9 µm resultiert. Die Platte wird unter einer 5-kW-Metallhalogenidlampe im Abstand von 110 cm 30 s lang durch einen Halbtonstufenkeil mit 13 Stufen einer Dichteabstufung von 0,15 belichtet, 1 min bei 100 °C nacherwärmt und in einem wäßrig-alkalischen Entwickler der im Beispiel 19 angegebenen Zusammensetzung 30 s lang entwickelt. Mit der so erhaltenen Positiv-Druckform werden auf einer Offsetdruckmaschine über 90.000 Drucke in guter Qualität erhalten.

### Beispiele 29 bis 32

Diese Beispiele zeigen die Verringerung des Dunkelabtrags von Kopierschichten bei gleichzeitig unveränderter Lichtempfindlichkeit durch den Zusatz der erfindungsgemäßen Verbindungen.

Platten aus elektrochemisch aufgerauhtem und anodisierten Aluminium werden mit einer Lösung aus
- 8,00 Gt: Bindemittel, erhalten durch Umsetzung von 2 Gt Polyhydroxystyrol (MW 4500) mit 1 Gt Di-tert-butyldicarbonat,
- 1,00 Gt: N,N-disubstituiertem Sulfonamid,
- 0,50 Gt: Säurespender 2,5-Diethoxy-4-(p-tolylmercapto)-benzoldiazoniumhexafluorphosphat,
- 0,08 Gt: Kristallviolettbase und
- 175 Gt: Methylethylketon
schleuderbeschichtet und bei 100 °C im Umlufttrockenschrank getrocknet, wobei Schichtdicken von 1,9 µm resultieren. Die Platten wurden unter einer 5 kW Metallhalogenidlampe im Abstand von 110 cm durch einen Halbtonstufenkeil mit 13 Stufen einer Dichteabstufung von 0,15 belichtet, 1 min bei 100 °C nacherwärmt und in einem wäßrig-alkalischen Entwickler der im Beispiel 19 angegebenen Zusammensetzung entwickelt, wobei die Platten einmal 30 s, ein anderes Mal jedoch 270 s im Entwickler verblieben. Die Ergebnisse zeigt Tabelle 3.

**Tabelle 3**

| Verringerung des Dunkelabtrags von Kopierschichten durch die erfindungsgemäßen Verbindungen | | | | | |
|---|---|---|---|---|---|
| Beispiel | Sulfonamid lt. Tab. 1 | offene Halbtonkeilstufe | | Linien in µ | |
| | | 30 s | 270 s | 30 s | 270 s |
| 29 | 1 | 3 | 5 | 15 | 20 |
| 30 | 4 | 3 | 4 | 15 | 20 |
| 31 | 2 | 3 | 5 | 15 | 20 |
| 32 | 3 | 3 | 5 | 15 | 20 |
| Vgl.* | - | 3 | 6 | 15 | 25 |

| | | | | | |
|---|---|---|---|---|---|
| * In dieser Schicht (Referenzschicht) ist der Anteil an erfindungsgemäßer Verbindung durch einen entsprechend höheren Bindemittelanteil ersetzt. | | | | | |

## Patentansprüche

1. N,N-disubstituierte Sulfonamide der allgemeinen Formeln R¹[-N(CO-OR²)-SO₂-R³]ₙ (I) oder R¹[-SO₂-N(CO-OR²)-R³]ₙ (II), worin
R¹ der n-wertige Rest eines (C₁-C₂₀)Alkans oder (C₅-C₇)Cycloalkans, wobei in den n-wertigen (C₃-C₂₀)Alkanresten einzelne Methylengruppen durch Heteroatome, durch (C₅-C₇)Cycloalkylenreste oder durch Phenylen ersetzt sein können, oder der n-wertige Rest eines nicht-kondensierten oder kondensierten ein- oder mehrkerniger (C₆-C₁₈)Aromaten,
R² ein (C₃-C₁₁)Alkyl-, (C₃-C₁₁)Alkenyl- oder (C₇-C₁₁)Aralkylrest,
R³ ein unsubstituierter oder substituierter (C₁-C₆)Alkyl-, (C₃-C₆)Cycloalkyl-, (C₆-C₁₄)Aryl- oder (C₇-C₂₀)Aralkylrest
ist, und
n eine ganze Zahl von 2 bis 4 bedeutet.

2. Sulfonamide gemäß Anspruch 1, dadurch gekennzeichnet, daß in dem Rest R¹ nicht mehr als drei Methylengruppen durch Heteroatome, insbesondere Sauerstoffatome, durch Cycloalkylen und/oder Phenylen ersetzt sind.

3. Sulfonamide gemäß Anspruch 1 oder 2, dadurch gekennzeichnet, daß n = 2 ist.

4. Sulfonamide gemäß Anspruch 1, dadurch gekennzeichnet, daß der Rest R¹ im Fall n = 2 die Formel - A - X - B - hat, worin X eine C-C-Einfachbindung, eine Methylengruppe oder ein Sauerstoffatom darstellt und A und B, unabhängig voneinander, für Arylen, insbesondere ortho-, meta- oder para-Phenylen, oder für Cycloalkandiyl, insbesondere Cyclohexandiyl, stehen.

5. Sulfonamide gemäß Anspruch 1, dadurch gekennzeichnet, daß R² ein Isopropyl-, sec.-Butyl- oder tert.-Butylrest ist.

6. Strahlungsempfindliches Gemisch mit
a) einer Verbindung, die unter der Einwirkung aktinischer Strahlung Säure bildet,
b) einer säurespaltbaren Verbindung, deren Spaltprodukte in einem wäßrig-alkalischen Entwickler eine höhere Löslichkeit zeigen als die Ausgangsverbindung, und
c) einem in Wasser unlöslichen, in einem wäßrig-alkalischen Entwickler dagegen löslichen, zumindest quellbaren, polymeren, organischen Bindemittel,
dadurch gekennzeichnet, daß die säurespaltbare Verbindung b) ein N,N-disubstituiertes Sulfonamid der allgemeinen Formeln R¹[-N(CO-OR²)-SO₂-R³]ₙ (I) oder R¹[-SO₂-N(CO-OR²)-R³]ₙ (II) ist, worin
R¹ im Fall n = 1, ein (C₁-C₂₀)Alkyl-, (C₃-C₁₀)Cycloalkyl-, (C₆-C₁₄)Aryl- oder (C₇-C₂₀)Aralkylrest, wobei in den Alkyl enthaltenden Resten einzelne Methylengruppen durch Heteroatome ersetzt sein können, und
im Fall n = 2, 3 oder 4, der n-wertige Rest eines (C₁-C₂₀)Alkans oder (C₅-C₇)Cycloalkans, wobei in den n-wertigen (C₃-C₂₀)Alkanresten einzelne Methylengruppen durch Heteroatome, durch (C₅-C₇)Cycloalkylenreste oder durch Phenylen ersetzt sein können, oder der n-wertige Rest eines nicht-kondensierten oder kondensierten ein- oder mehrkerniger (C₆-C₁₈)Aromaten,
R² ein (C₃-C₁₁)Alkyl-, (C₃-C₁₁)Alkenyl- oder (C₇-C₁₁)Aralkylrest,
R³ ein unsubstituierter oder substituierter (C₁-C₆)Alkyl-, (C₃-C₆)Cycloalkyl-, (C₆-C₁₄)Aryl- oder (C₇-C₂₀)Aralkylrest
ist, und
n eine ganze Zahl von 1 bis 4 bedeutet.

7. Strahlungsempfindliches Gemisch gemäß Anspruch 6, dadurch gekennzeichnet, daß der Anteil der säurebildenden Verbindung(en) a) 0,1 bis 20 Gew.-%, bevorzugt 0,2 bis 10% Gew.-%, jeweils bezogen auf das Gesamtgewicht der Feststoffe in dem Gemisch, beträgt.

8. Strahlungsempfindliches Gemisch gemäß Anspruch 6, dadurch gekennzeichnet, daß der Anteil der säurespaltbaren Verbindung(en) b) 5 bis 50 Gew.-%, bevorzugt 10 bis 30 Gew.-%, jeweils bezogen auf das Gesamtgewicht der Feststoffe in dem Gemisch beträgt.

9. Strahlungsempfindliches Gemisch gemäß Anspruch 6, dadurch gekennzeichnet, daß der Anteil des polymeren Bindemittels c) 30 bis 90 Gew.-%, bevorzugt 55 bis 85 Gew.-%, jeweils bezogen auf das Gesamtgewicht der Feststoffe in dem Gemisch, beträgt.

10. Aufzeichnungsmaterial mit einem Träger und einer strahlungsempfindlichen Schicht, dadurch gekennzeichnet, daß die Schicht aus einem strahlungsempfindlichen Gemisch gemäß einem oder mehreren der Ansprüche 6 bis 9 besteht.

## Claims

1. N,N-Disubstituted sulfonamides of the formulae
R¹[-N(CO-OR²)-SO₂-R³]ₙ (I) or
R¹[-SO₂-N(CO-OR²)-R³]ₙ (II),
in which
R¹ is the n-valent radical of a (C₁-C₂₀)alkane or (C₅-C₇)cycloalkane, individual methylene groups in the n-valent (C₃-C₂₀)alkane radicals being optionally replaced by heteroatoms, by (C₅-C₇)cycloalkylene radicals or by phenylene, or is the n-valent radical of a noncondensed or condensed mono- or polynuclear (C₆-C₁₈)aromatic compound,
R² is a (C₃-C₁₁)alkyl, (C₃-C₁₁)alkenyl or (C₇-C₁₁)aralkyl radical,
R³ is an unsubstituted or substituted (C₁-C₆)alkyl, (C₃-C₆)cycloalkyl, (C₆-C₁₄)aryl or (C₇-C₂₀)aralkyl radical, and
n is an integer from 2 to 4.

2. Sulfonamides as claimed in claim 1, wherein in the radical R¹ not more than three methylene groups are replaced by heteroatoms, in particular oxygen atoms, by cycloalkylene and/or phenylene.

3. Sulfonamides as claimed in claim 1 or 2, wherein n = 2.

4. Sulfonamides as claimed in claim 1, wherein the radical R¹ for n = 2 has the formula -A-X-B-, in which X is a C-C single bond, a methylene group or an oxygen atom, and A and B, independently of one another, are arylene, in particular ortho-, meta- or para-phenylene, or cycloalkanediyl, in particular cyclohexanediyl.

5. Sulfonamides as claimed in claim 1, wherein R² is an isopropyl, sec-butyl or t-butyl radical.

6. A radiation-sensitive mixture which comprises
a) a compound which under the influence of actinic radiation forms acid,
b) an acid-cleavable compound whose cleavage products in an aqueous-alkaline developer have a higher solubility than the starting compound, and
c) a polymeric organic binder which is insoluble in water but is soluble or at least swellable in an aqueous alkaline developer
wherein the acid-cleavable compound b) is an N,N-disubstituted sulfonamide of the general formulae R¹[-N(CO-OR²)-SO₂-R³]ₙ (I) or R¹[-SO₂-N(CO-OR²)-R³]ₙ (II), in which
R¹ for n = 1 is a (C₁-C₂₀)alkyl, (C₃-C₁₀)cycloalkyl, (C₆-C₁₄)aryl or (C₇-C₂₀)aralkyl radical, individual methylene groups in the radicals containing alkyl being optionally replaced by heteroatoms,
for n = 2, 3 or 4 the n-valent radical of a (C₁-C₂₀)alkane or (C₅-C₇)cycloalkane, individual methylene groups in the n-valent (C₃-C₂₀)alkane radicals being optionally replaced by heteroatoms, by (C₅-C₇)cycloalkylene radicals or by phenylene, or is the n-valent radical of a noncondensed or condensed mono- or polynuclear (C₆-C₁₈)aromatic compound,
R² is a (C₃-C₁₁)alkyl, (C₃-C₁₁)alkenyl or (C₇-C₁₁)aralkyl radical,
R³ is an unsubstituted or substituted (C₁-C₆)alkyl, (C₃-C₆)cycloalkyl, (C₆-C₁₄)aryl or (C₇-C₂₀)aralkyl radical, and
n is an integer from 1 to 4.

7. The radiation-sensitive mixture as claimed in claim 6, wherein the proportion of the acid-forming compound(s) a) is from 0.1 to 20% by weight, preferably from 0.2 to 10% by weight, in each case based on the total weight of the solids in the mixture.

8. The radiation-sensitive mixture as claimed in claim 6, wherein the proportion of the acid-cleavable compound(s) b) is from 5 to 50% by weight, preferably from 10 to 30% by weight, in each case based on the total weight of the solids in the mixture.

9. The radiation-sensitive mixture as claimed in claim 6, wherein the proportion of the polymeric binder c) is from 30 to 90% by weight, preferably from 55 to 85% by weight, in each case based on the total weight of the solids in the mixture.

10. A recording material comprising a support and a radiation-sensitive layer, wherein the layer comprises a radiation-sensitive mixture according to one or more of claims 6 to 9.

## Revendications

1. Sulfonamides N,N-disubstitués de formule générale
R¹[-N(CO-OR²)-SO₂-R³]ₙ (I) ou
R¹[-SO₂-N(CO-OR²)-R³]ₙ (II),
formules dans lesquelles
R¹ représente le reste n-valent d'un alcane en C₁-C₂₀ ou d'un cycloalcane en C₅-C₇, des groupes méthylène individuels dans les restes n-valents d'alcanes en C₃-C₂₀ pouvant être remplacés par des hétéroatomes, par des radicaux cycloalkylène en C₅-C₇ ou par le radical phénylène, ou le reste n-valent d'un composé aromatique en C₆-C₁₈ mono- ou polynucléaire, condensé ou non condensé,
R² représente un radical alkyle en C₃-C₁₁, alcényle en C₃-C₁₁ ou aralkyle en C₇-C₁₁,
R³ représente un radical alkyle en C₁-C₆, cycloalkyle en C₃-C₆, aryle en C₆-C₁₄ ou aralkyle en C₇-C₂₀, substitué ou non substitué, et
n représente un nombre entier allant de 2 à 4.

2. Sulfonamides selon la revendication 1, caractérisés en ce que, dans le radical R¹, il n'y a pas plus de trois groupes méthylène remplacés par des hétéroatomes, en particulier des atomes d'oxygène, ou par le radical cycloalkylène et/ou le radical phénylène.

3. Sulfonamides selon la revendication 1 ou 2, caractérisés en ce que n est égal à 2.

4. Sulfonamides selon la revendication 1, caractérisés en ce que le radical R¹, lorsque n = 2, correspond à la formule -A-X-B-, dans laquelle X représente une liaison simple C-C, un groupe méthylène ou un atome d'oxygène, et A et B, indépendamment l'un de l'autre, représentent un groupe arylène, en particulier o-, m- ou p-phénylène, ou un radical cycloalcanediyle, en particulier cyclohexanediyle.

5. Sulfonamides selon la revendication 1, caractérisés en ce que R² est un radical isopropyle, sec-butyle ou tert-butyle.

6. Composition sensible aux radiations, comportant
a) un composé qui forme un acide sous l'effet d'un rayonnement actinique,
b) un composé dissociable par un acide, dont les produits de coupure présentent une plus grande solubilité dans un révélateur aqueux-alcalin que le composé de départ, et
c) un liant organique polymère, insoluble dans l'eau, mais soluble, ou au moins susceptible de gonfler, dans un révélateur aqueux-alcalin,
caractérisée en ce que le composé b) dissociable par un acide est un sulfonamide N,N-disubstitué de formule générale
R¹[-N(CO-OR²)-SO₂-R³]ₙ (I) ou
R¹[-SO₂-N(CO-OR²)-R³]ₙ (II),
formules dans lesquelles
R¹ représente, lorsque n = 1, un radical alkyle en C₁-C₂₀, cycloalkyle en C₃-C₁₀, aryle en C₆-C₁₄ ou aralkyle en C₇-C₂₀, des groupes méthylène individuels dans les radicaux contenant des fractions alkyle pouvant être remplacés par des hétéroatomes, et
représente, lorsque n = 2, 3 ou 4, le reste n-valent d'un alcane en C₁-C₂₀ ou d'un cycloalcane en C₅-C₇, des groupes méthylène individuels dans les restes n-valents d'alcanes en C₃-C₂₀ pouvant être remplacés par des hétéroatomes, par des radicaux cycloalkylène en C₅-C₇ ou par le radical phénylène, ou le reste n-valent d'un composé aromatique en C₆-C₁₈ mono- ou polynucléaire, condensé ou non condensé,
R² représente un radical alkyle en C₃-C₁₁, alcényle en C₃-C₁₁ ou aralkyle en C₇-C₁₁,
R³ représente un radical alkyle en C₁-C₆, cycloalkyle en C₃-C₆, aryle en C₆-C₁₄ ou aralkyle en C₇-C₂₀, substitué ou non substitué, et
n représente un nombre entier allant de 1 à 4.

7. Composition sensible aux radiations selon la revendication 6, caractérisée en ce que la proportion du(des) composé(s) a) générateur(s) d'acide va de 0,1 à 20 % en poids, de préférence de 0,2 à 10 % en poids, dans chaque cas par rapport au poids total des substances solides dans la composition.

8. Composition sensible aux radiations selon la revendication 6, caractérisée en ce que la proportion du(des) composé(s) b) dissociable(s) par un acide va de 5 à 50 % en poids, de préférence de 10 à 30 % en poids, dans chaque cas par rapport au poids total des substances solides dans la composition.

9. Composition sensible aux radiations selon la revendication 6, caractérisée en ce que la proportion du liant polymère c) va de 30 à 90 % en poids, de préférence de 55 à 85 % en poids, dans chaque cas par rapport au poids total des substances solides dans la composition.

10. Matériau de reprographie comportant un support et une couche sensible aux radiations, caractérisé en ce que la couche est formée à partir d'une composition sensible aux radiations selon une ou plusieurs des revendications 6 à 9.
